# EUROPEAN PATENT SPECIFICATION

(11) **EP 3 190 971 B1**
(45) Date of publication and mention of the grant of the patent: **05.06.2019**
(21) Application number: 15766723.9
(22) Date of filing: 10.09.2015
(51) Int. Cl.: A61B 5/154, A61B 5/155, A61B 5/15, A61L 29/08, A61L 29/16

(54) **BLOOD SAMPLING SYSTEM FOR IMPROVING DRAW SUCCESS AND REDUCING HEMOLYSIS**
BLUTENTNAHMESYSTEM ZUR VERBESSERUNG DES ENTNAHMEERFOLGS UND ZUR REDUZIERUNG VON HÄMOLYSE
SYSTÈME DE PRÉLÈVEMENT SANGUIN PERMETTANT D'AMÉLIORER LA RÉUSSITE DU PRÉLÈVEMENT ET DE RÉDUIRE L'HÉMOLYSE

(30) Priority: 11.09.2014 US 201414483780
(43) Date of publication of application: 19.07.2017
(73) Proprietor: Becton, Dickinson and Company, Franklin Lakes, NJ 07417-1880 (US)
(72) Inventor: BURKHOLZ, Jonathan Karl, Salt Lake City, Utah 84108 (US); MCKINNON, Austin Jason, Herriman, Utah 84096 (US); ADAMS, Chad M., Cedar Hills, Utah 84062 (US)
(74) Representative: dompatent von Kreisler Selting Werner - Partnerschaft von Patent- und Rechtsanwälten mbB
(86) International application number: PCT/US2015/049473
(87) International publication number: WO 2016/040668

(56) References cited:
- EP-A2- 0 555 109
- WO-A1-2008/038012
- US-A- 4 140 108
- US-A- 4 703 762
- US-A1- 2009 131 769
- US-B2- 8 353 876
- US-B2- 8 383 044
- US-B2- 8 403 911

## Description

### BACKGROUND OF THE INVENTION

The present invention relates generally to a blood sampling system for improving blood draw success and reducing hemolysis within the blood sample. More particularly, the present invention is directed to intravascular systems such as peripheral intravenous catheters, blood collection sets, peripherally inserted central catheters, etc. that are configured to optimize the performance of the systems when used to draw blood samples.

Blood samples are increasingly being taken using peripheral intravenous catheters that have been designed primarily for fluid injection. Although such peripheral intravenous catheters can be used to obtain blood samples, they are oftentimes ineffective or produce blood samples that are inadequate. For example, these catheters can often experience flow restrictions due to various factors including the extra-dermal or sub-dermal kinking of the catheter, the blockage of the catheter tip against a vein wall or valve, and the blockage of the catheter tip or lumen due to indwelling factors such as clotting. When flow restrictions exist, the clinician can be prevented from obtaining a sufficient amount of blood to perform the desired testing.

Further, even if a sufficient quantity of blood can be withdrawn using existing peripheral intravenous catheters, the design of the catheter and/or the presence of flow restrictions within the catheter can affect the quality of the blood. For example, as blood is withdrawn through existing peripheral intravenous catheters, a substantial amount of hemolysis may be caused making the blood sample unsuitable for many tests.

US 8,353,876 B2 discloses a device for preventing an occlusion of a catheter including a catheter adapter and a catheter where a flexured portion of the catheter is supported by a bending surface of the catheter adapter.

US 8,403,911 B2 discloses a peripheral catheter having a catheter tip diffuser for reducing an exit velocity of an infusant within the catheter.

US 8,383,044 B2 discloses a blood sampling device useful for collecting a blood sample from a separate vascular access device.

### BRIEF SUMMARY OF THE INVENTION

The invention provides an intravenous system comprising: a catheter adapter; catheter tubing extending from the catheter adapter; a kink resistant feature that prevents the catheter tubing from kinking; and a blood sampling device configured to collect a small blood sample for point-of-care testing and a large blood sample for laboratory testing; wherein the small blood sample is collected within a reservoir of the blood sampling device, and the large blood sample is collected within a sample container connected to the blood sampling device via a sample container collection device.

The present invention extends to intravenous systems that are optimized to improve blood draw success and reduce hemolysis within the blood sample. Multiple optimizations can be made to an intravenous system, such as a peripheral intravenous catheter, to enhance the system's ability to provide blood samples having sufficient quality for many different tests.

These optimizations can include features which enable an indwelling system, such as a peripheral intravenous catheter, to continue to perform efficiently when used to obtain blood samples even after the system has been placed within the patient's vasculature for a substantial duration of time. Also, these optimizations can include features for optimizing the fluid path and flow characteristics during blood draw to minimize the amount of hemolysis that may be caused during blood draw. Further, these optimizations can include features for integrating blood acquisition and dispense capabilities within the system, including features for making the system compatible with large blood sample collection methods.

Each of the various optimizations can be used singly or in combination to enhance the performance of an indwelling system when used for obtaining blood samples. Suitable indwelling systems that can be enhanced in accordance with the present invention include peripheral intravenous catheters whether integrated or non-integrated or with or without an extension set, central venous catheters, and peripherally inserted central catheters. These optimizations can also be used in other (i.e. non-indwelling) systems to increase their ability to obtain quality blood samples. For example, one or more of the optimizations can be used to optimize a blood collection set.

When two or more of the various optimizations are used in combination, additional benefits can be obtained. For example, although a single optimization can enhance the ability of a peripheral intravenous catheter to be used for blood sampling, without a combination of enhancements, the catheter may not function in an optimal manner. The present invention therefore offers various combinations of features that can ensure optimal performance of an intravenous system by minimizing the occurrence of fluid pathway occlusions or other performance degradations that would otherwise reduce the amount and/or quality of blood obtained from the device.

The present invention is implemented as an intravenous system as defined in claim 1.

This summary is provided to introduce a selection of concepts in a simplified form that are further described below in the Detailed Description. This Summary is not intended to identify key features or essential features of the claimed subject matter.

Additional features and advantages of the invention will be set forth in the description which follows, and in part will be obvious from the description, or may be learned by the practice of the invention. The features and advantages of the invention may be realized and obtained by means of the instruments and combinations particularly pointed out in the appended claims. These and other features of the present invention will become more fully apparent from the following description and appended claims, or may be learned by the practice of the invention as set forth hereinafter.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figure 1 is a perspective view illustrating an example peripheral intravenous catheter that can be optimized to include one or more features for increasing the catheter's ability to collect blood in accordance with one or more embodiments of the invention;
Figure 2 is a cross-sectional view of a diffuser tip that can be employed on catheter tubing of an intravenous system to increase blood flow into the catheter tubing;
Figures 3A and 3B are perspective and cross-sectional views respectively illustrating an example antimicrobial coating that can be used as a kink resistant feature on catheter tubing; and
Figures 4A and 4B are cross-sectional views illustrating an example blood sampling device that can be attached to an intravenous system to allow the collection of small and large blood samples.

### DETAILED DESCRIPTION OF THE INVENTION

The following description will present various optimizations that can be employed on an intravenous system in accordance with one or more embodiments of the invention. Any combination of the described optimizations may be used to provide an intravenous system that is optimized for blood sampling. Although the following description will present these optimizations with reference to a peripheral intravenous catheter, corresponding optimizations can be made to other types of intravenous systems.

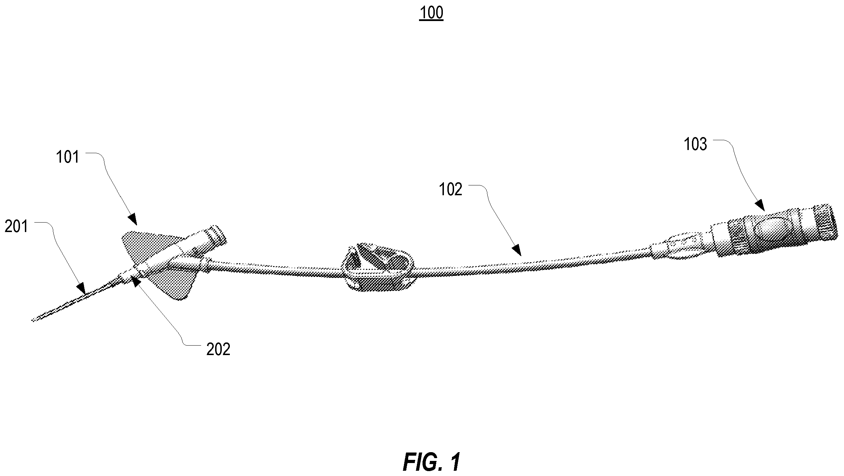

Figure 1 depicts an example peripheral intravenous catheter ("PIVC") 100 in accordance with one or more embodiments of the present invention. PIVC 100 generally comprises a catheter 101 comprised of catheter tubing 201 and a catheter adapter 202, extension tubing 102, and a blood sampling device 103 that is attached to a proximal end of extension tubing 102. As stated in the previous paragraph, a PIVC configured with components different from those shown in Figure 1 as well as other types of vascular access devices could be optimized with any combination of the following features in accordance with the present invention. For example, in some embodiments, a PIVC may not include extension tubing 102 or blood sampling device 103. Figure 1 therefore serves only as a representation of one system that can be optimized in accordance with present invention.

### Diffuser Tip For Catheter Tubing

In some embodiments of the invention, PIVC 100 can include a diffuser tip that is configured to provide redundant pathways for flow of blood into the catheter. Example embodiments of a diffuser tip that can be employed in embodiments of the invention are described in United States Patent No. 8,403,911, titled SYSTEMS AND METHODS FOR IMPROVING CATHETER HOLE ARRAY EFFICIENCY ("the '911 patent").

The '911 patent describes the use of a diffuser tip for reducing the exit velocity of a fluid when a peripheral intravenous catheter is used to inject the fluid. Rather than employing a diffuser tip to reduce the exit velocity, PIVC 100, in accordance with embodiments of the present invention, can be configured with a diffuser tip to prevent the catheter tip from becoming restricted during blood draw. During typical usage of a peripheral intravenous catheter (i.e. during fluid infusion), blockage of the catheter tip by a vein wall or other structure within the vasculature is generally not a concern because the exiting fluids can create a sufficient gap between the catheter tip and any nearby structure to allow the fluid to exit the catheter. During blood draw, however, the suction forces that are created at the catheter tip can encourage the catheter tip to become partially or completely blocked by nearby structures. The use of a diffuser tip can therefore create multiple fluid pathways through which blood can enter the catheter, and can provide multiple benefits when a peripheral intravenous catheter is used as a blood sampling system. For example, by providing multiple pathways, the diffuser tip minimizes the likelihood of the catheter tip becoming restricted and also minimizes the pressure experienced at the catheter tip. Further, the multiple pathways enable a greater flow rate of blood into the catheter. Each of these benefits can also minimize the occurrence of hemolysis in the sampled blood.

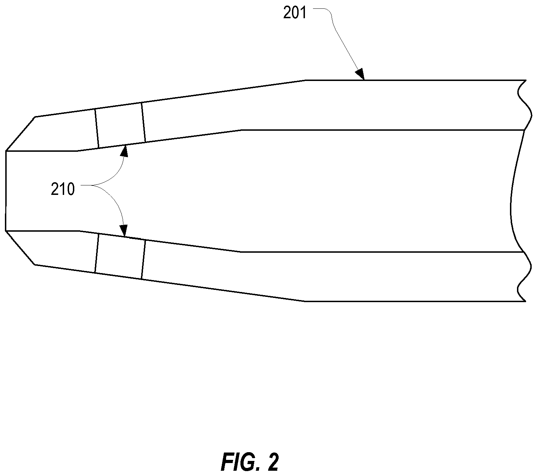

Accordingly, catheter tubing 201 can be configured with a diffuser tip to enhance the performance of PIVC 100 when drawing blood. In some embodiments, the diffuser tip can include at least two holes in addition to the distal opening. Figure 2 provides an example where catheter tubing 201 includes a diffuser tip comprised of two holes 210. Holes 210 can be positioned on opposite sides of the diffuser tip. By positioning the holes on opposite sides, it is less likely that both holes will become blocked. For example, if the diffuser tip is positioned near the vein wall during blood draw, the suction from the blood draw may cause the diffuser tip to contact the vein wall plugging one of the holes. However, because the other hole is positioned on the opposite side of the diffuser tip, the flow of blood may continue through the opposite hole thereby ensuring that an adequate flow continues until the blood sample is obtained.

In some embodiments, such as when catheter adapter 102 includes a base for orienting the catheter adapter (and as a result, orienting the catheter tubing), the holes can be formed in a particular orientation. For example, with the catheter adapter oriented in its intended position, two holes may be positioned on a top and bottom of the diffuser tip or on a left and right side of the diffuser tip. The particular orientation may be based on the intended location where the catheter will be inserted. For example, if PIVC 100 is typically used in a location that generally causes the bottom of the diffuser tip to be positioned against the vein wall, holes 210 can be oriented on left and rights sides of the diffuser tip. Alternatively, if PIVC 100 is typically used in a location that generally causes a side of the diffuser tip to be positioned against the vein wall, holes 210 can be oriented on the top and bottom of the diffuser tip.

Although Figure 2 illustrates a diffuser tip having only two holes that are positioned on opposite sides, a diffuser tip having one or more holes may be used. Similarly, when more than one hole is used, the holes may be positioned in any suitable pattern including opposite one another as described above.

### Kink Resistant Features For Catheter Tubing

PIVC 100 can include an occlusion resistant catheter to minimize the likelihood that the catheter will become kinked during blood draw. Examples of suitable occlusion resistant catheters are described in United States Patent No. 8,353,876, titled OCCLUSION RESISTANT CATHETERS ("the '876 patent").

The '876 patent describes various features that can be employed on the catheter adapter or the catheter tubing to minimize kinking of the catheter tubing during use. These features include configuring the catheter adapter tip with a chamfer, a contour, and/or a step, and configuring the catheter tubing with a maximum insertion length mark, an external support sleeve, and/or an internal supportive coil. Any combination of these features described in the '876 patent can be employed in embodiments of the present invention to enhance the performance of PIVC 100 when used to collect blood.

In some embodiments, PIVC 100 can include an antimicrobial coating that functions as a strain relief feature. In other words, PIVC 100 can include an antimicrobial coating that functions both to release an antimicrobial agent and to provide external support to the catheter tubing to minimize kinking of the catheter tubing. Suitable embodiments of an antimicrobial coating that can function as a strain relief feature are described in United States Patent Application No. 14/326,036, titled ANTIMICROBIAL COATING FORMING KINK RESISTANT FEATURE ON A VASCULAR ACCESS DEVICE (the '036 application).

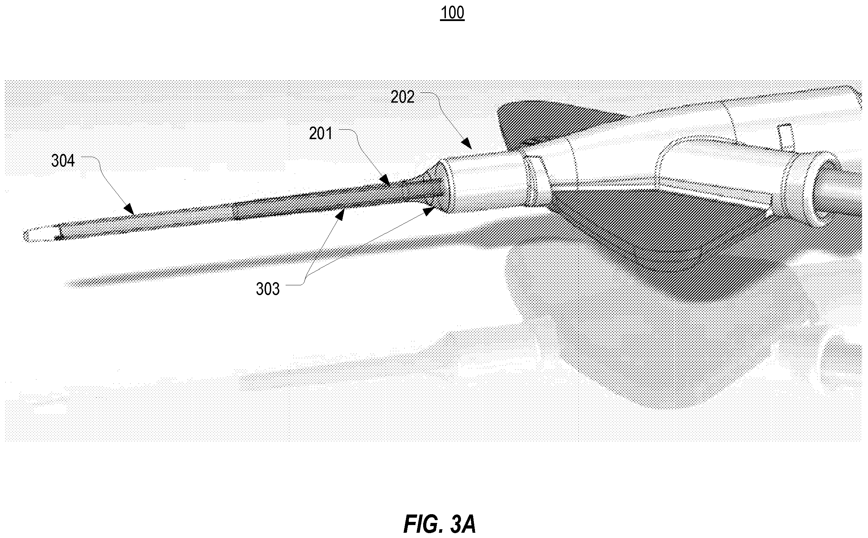

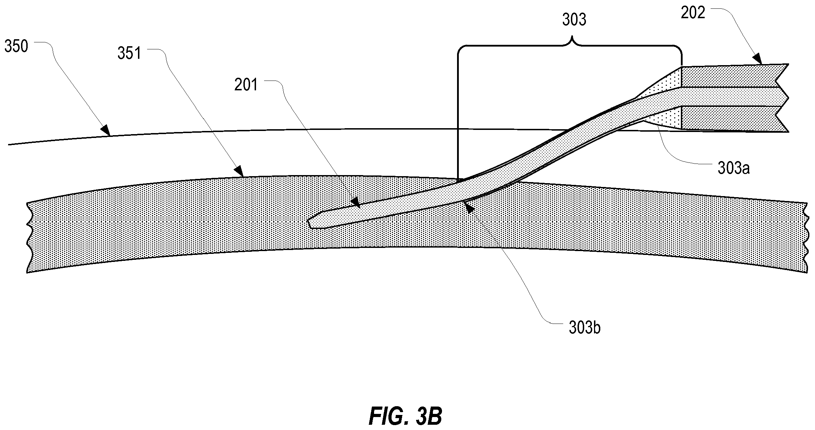

As described in the '036 application and as shown in Figures 3A and 3B, an antimicrobial coating 303 can be applied at the base of the catheter tubing 201 (i.e. where the catheter tubing 201 enters the catheter adapter 202) to form a supportive structure around the catheter tubing. The antimicrobial coating 303 can include an increased diameter portion 303a adjacent the catheter adapter 202 to increase the kink resistance of the catheter tubing as it exits the catheter adapter. As best shown in Figure 3B, increased diameter portion 303a provides additional kink resistance at the catheter adapter, the point where catheter tubing 201 is most likely to kink. The antimicrobial coating can be formed of a base (e.g. polymer) material and one or more antimicrobial agents which are released or eluted from the base material at a controlled rate. For example, the base material can be a water softening UV cure matrix such as UV cured acrylate-urethanes, or heat-cured polyurethanes.

In some embodiments, antimicrobial coating 303 can have a length that is based on a length of the catheter tubing that should remain outside the patient's skin 350. For example, this length of the antimicrobial coating can be configured such that the distal end 303b of the coating extends up to, if not slightly through, an intimal layer of a vein 351 in which the catheter is inserted as shown in Figure 3B. By configuring antimicrobial coating 303 with this length, antimicrobial agents can be targeted to the sub-dermal layers of skin where bacteria is most likely to reside while also providing kink resistance along the portion of catheter tubing 201 that is most likely to kink.

Antimicrobial coating 303 can therefore provide dual benefits of minimizing the occurrence of kinks and providing antimicrobial agents to a targeted region and at a controlled rate. Antimicrobial coating 303 can therefore be particularly beneficial when used on PIVC 100 or other intravenous systems that have typically been designed for infusion but are also used for blood draw. For example, when fluids are being injected, there is less concern for kinking because the pressure of the fluid tends to create adequate fluid flow even when a kink is present. Further, there is no concern for hemolysis in the injected fluid. However, when these same devices are employed to draw blood, a kink is more likely to minimize flow rates below an acceptable level because blood flow is dependent on the patient's blood pressure rather than an external source. Even if a kink allows blood to flow at an acceptable level, the kink may still cause hemolysis in the blood. Antimicrobial coating 303 can therefore be particularly beneficial to prevent kinks during blood draw.

When configured with any of the above described kink resistant features, PIVC 100 can exhibit a reduced tendency for occlusion of the fluid pathway resulting in greater blood draw rates and a reduced occurrence of hemolysis within blood samples. Therefore, the kink resistant features, especially when combined with one or more optimizations of the present invention, can enhance the functionality of PIVC 100 when used for blood sampling.

For example, if only a diffuser tip is used on PIVC 100, catheter tubing 201 may still become kinked. If a kink occurs, the diffuser tip will provide little or no benefit during blood draw because the flow of blood will already be limited by the kink and/or any blood obtained may be substantially hemolyzed. Similarly, if only a kink resistant feature is used, the catheter tip may become blocked thereby limiting or preventing the flow of blood into the catheter tubing. Accordingly, the combination of a kink resistant feature and a diffuser tip provides synergistic benefits to a PIVC or other intravenous system when used to collect blood.

### Coatings for Catheter Tubing To Reduce Occlusion Due To Clotting

In some embodiments of the present invention, a coating can be applied to catheter tubing 201 to reduce occlusions due to clotting. In contrast to antimicrobial coating 203 described above, these coatings can serve to prevent clotting rather than provide kink resistance. However, antimicrobial coating 203, in some embodiments, can assist in preventing clots such as when antimicrobial coating 203 extends along the full length of catheter tubing 201.

An example of a suitable antimicrobial coating that can be applied to catheter tubing 201 to prevent clots is disclosed in United States Patent No: 8,426,348, titled ANTIMICROBIAL LUBRICANT COMPOSITIONS ("the '348 patent"). The antimicrobial coatings described in the '348 patent can be used to provide lubrication to catheter tubing 201 as well as to minimize the occurrence of clots that may occlude one or more openings of catheter tubing 201 (e.g. holes 210 of the diffuser tip). Another example of a suitable antimicrobial coating that can be applied to catheter tubing 201 is a polyethylene oxide based coating.

Any of these coatings can be applied to a portion of catheter tubing 201 to prevent the occurrence of blood clots. In particular, these coatings can be used in conjunction with a diffuser tip to prevent the diffuser tip from becoming occluded. For example, an antimicrobial coating can be applied in and/or around holes 210 to prevent clots from forming in the holes. Accordingly, the use of an antimicrobial coating in combination with a diffuser tip can enhance the performance of PIVC 100 when used to collect blood. In some embodiments, additional benefits can be obtained by employing a combination of an antimicrobial coating, a diffuser tip, and a kink resistant feature.

An antimicrobial coating can also be beneficial when used in combination with a kink resistant feature even when a diffuser tip is not employed. For example, Figure 3A illustrates an embodiment where PIVC 100 includes antimicrobial coating 303 along a proximal portion of catheter tubing 201 and antimicrobial lubricant 304 along a distal portion of catheter tubing 201. Antimicrobial lubricant 304 can prevent clots from forming that may occlude the distal opening of catheter tubing 201 while antimicrobial coating 303 can provide kink resistance.

### Catheter Tubing Material

In some embodiments of the present invention, the catheter tubing of PIVC 100 can be formed of a material that minimizes the likelihood of kinking and the formation of catheter-related thrombosis during indwelling periods. A material can also be used that minimizes the occurrence of phlebitis and the resulting catheter tip restriction that may otherwise occur. An example of a suitable material is polyurethane because it is water softening. As described above, antimicrobial coating 303 can also be formed of a water softening urethane material. Antimicrobial coating 303 can therefore be used on a polyurethane catheter tubing without substantially reducing the water softening characteristics of the catheter tubing.

### Blood Sampling Devices

In some embodiments of the present invention, PIVC 100 can be configured to incorporate a small blood sampling device 103 for collecting a sufficient amount of blood to perform point-of-care ("POC") testing. Examples of suitable small blood sampling devices that can be used with PIVC 100 are described in United States Patent No. 8,383,044, titled BLOOD SAMPLING DEVICE ("the '044 patent").

As described in the '044 patent, a blood sampling device can comprise a body having an internal chamber. The body can be shaped and sized to be connected to PIVC 100 (or another system) in various ways including, for example, via a port on extension set 102 or directly to a port on catheter adapter 202.

PIVC 100 includes a blood sampling device 103 that comprises a reservoir for collecting a small blood sample for POC testing and an adapter for connecting a sample container collection device for collecting a large blood sample for laboratory testing. Examples of suitable blood sampling devices that can be used for POC testing as well as for collecting large blood samples are disclosed in United States Patent Application No: 14/251,672, titled MEDICAL DEVICE FOR COLLECTION OF A BIOLOGICAL SAMPLE.

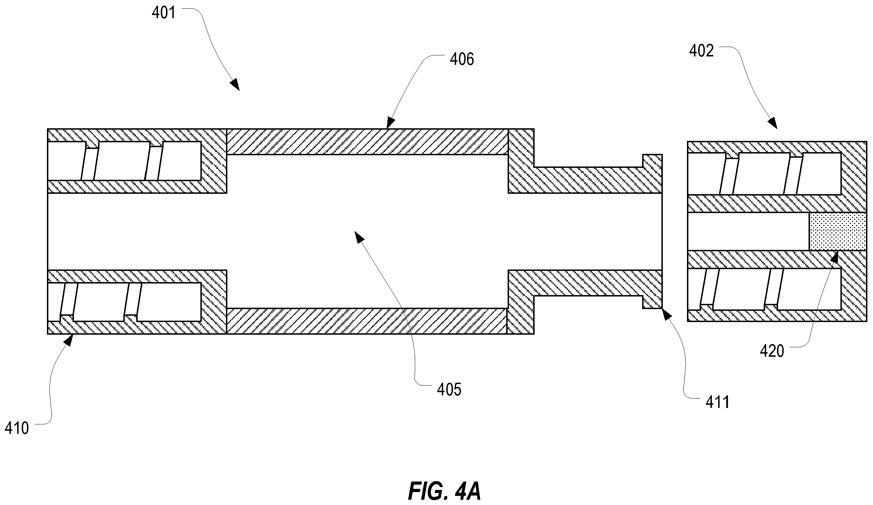

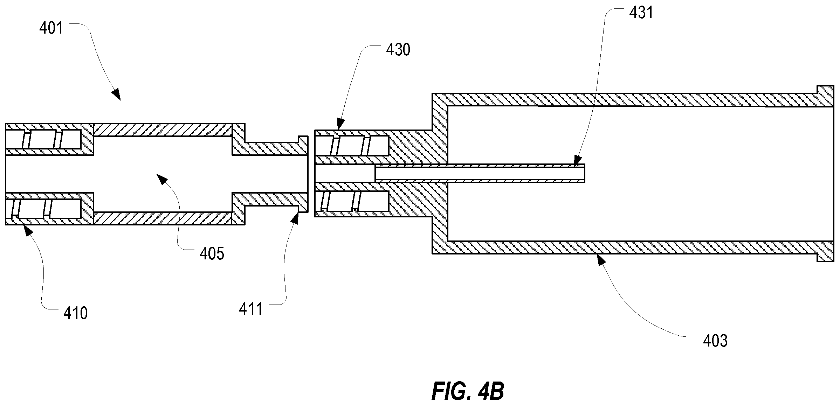

As described in the '672 application and as shown in the cross-sectional exploded view of Figures 4A and 4B, a blood sampling device 401 can comprise a first end 410 that is configured to connect to PIVC 100 and a second end 411 to which an end cap 402 (shown in Figure 4A) or a sample container collection device 403 (shown in Figure 4B) can be attached. First end 410 is shown as comprising a male luer lock connector. However, any other suitable connector could be used to attach blood sampling device 401 to a port of PIVC 100 whether via a direct connection to catheter adapter 202 or via extension tubing 102. Second end 411 is shown as comprising a female luer lock connector. However, as with first end 410, any other suitable connector could be used for second end 411.

Blood sampling device 401 comprises a reservoir 405 within which blood may flow and accumulate. Reservoir 405 can be used to collect a small blood sample for use in POC testing. In such cases, after blood is collected within reservoir 405, blood sampling device 401 can be detached from PIVC 100 to allow blood to be expelled from reservoir 405 onto a POC testing device. In some embodiments, blood sampling device 401 may include a compressible portion 406 to assist in expelling blood from reservoir 405. Also, in some embodiments, compressible portion 406 may be employed to pump blood from PIVC 100 into reservoir 405.

End cap 402 can be employed to seal second end 411 from an external environment. For example, end cap 402 can include venting material 420 that is configured to allow the passage of air but prevent the passage of blood. End cap 402 can therefore assist the flow of blood into reservoir 405.

As shown in Figure 4B, second end 411 is configured to allow sample container collection device 403 to be attached to blood sampling device 401 so that blood sampling device 401 can be used to obtain larger samples of blood. For example, sample container collection device 403 can be used in conjunction with one or more standard vacuum containers for collecting blood samples for laboratory testing.

Sample container collection device 403 includes a first end 430 that is configured to attach to second end 411 of blood sampling device 401. In the example shown in Figure 4B, second end 430 comprises a male luer lock connector. Sample container collection device 403 also includes a cannula 431 for piecing a seal of a sample container (not shown) when the sample container is inserted within sample container collection device 403. In some embodiments, cannula 430 may include a flow restrictor (not shown) such as a septum. The flow restrictor can be configured to open a flow path when a sample container is inserted within sample container collection device 403.

When sample container collection device 403 is used, both small and large blood samples can be obtained simultaneously. In other words, with sample container collection device 403 connected to blood sampling device 401, blood will flow through reservoir 405 into a sample container attached to sample container collection device 403. Once the sample container is sufficiently full, the sample container can be removed from sample container collection device 403 and sent to a lab for testing. Blood sampling device 401, while remaining attached to the sample container collection device 403, can be removed from the PIVC 100 to eject blood within the reservoir 405 for POC testing.

Accordingly, PIVC 100 includes a blood sampling device for collecting a small blood sample for POC testing. In some embodiments, the blood sampling device can also be configured to serve as an adapter for connecting a sample container collection device to PIVC 100 thereby facilitating the collection of large blood samples with PIVC 100.

When used in combination with any of the above described optimizations, a blood sampling device can further optimize a PIVC or other intravenous system for performing blood draws. For example, by employing a diffuser tip and a kink resistant feature on a PIVC that includes a blood sampling device, the flow of blood into the blood sampling device can be maintained so that an adequate quantity of blood can be collected without significant hemolysis. Without such optimizations, a kink or other occlusion may occur thereby minimizing the effectiveness of the blood sampling device.

Further, because a PIVC provides ready access to a patient's vasculature, employing a blood sampling device that enables the collection of large blood samples via the PIVC facilitates the collection of such samples. In other words, once a PIVC has been inserted into a patient's vasculature, a blood sample, whether for POC testing, laboratory testing, or both, can be obtained quickly and without inserting another device into the patient's vasculature. For PIVCs or other in-dwelling systems, the use of antimicrobial coating on the catheter tubing can provide the additional benefit of preventing clotting while the system is in place. Accordingly, a system that employs multiple or all of the above described optimizations can provide an enhanced means for collecting blood samples.

The present invention may be embodied in other specific forms without departing from its scope. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. An intravenous system comprising:
a catheter adapter (202);
catheter tubing (201) extending from the catheter adapter;
a kink resistant feature that prevents the catheter tubing from kinking; and
a blood sampling device (401) configured to collect a small blood sample for point-of-care testing and a large blood sample for laboratory testing;
**characterized in that**
the small blood sample is collected within a reservoir (405) of the blood sampling device, and the large blood sample is collected within a sample container connected to the blood sampling device via a sample container collection device (403).

2. The intravenous system of claim 1, wherein the catheter tubing has a diffuser tip.

3. The intravenous system of claim 2, wherein the diffuser tip includes an antimicrobial coating.

4. The intravenous system of claim 2, wherein
said blood sampling device has a first end that is configured to connect the blood sampling device to the catheter adapter directly or via an extension set (102), and a second end forming an adapter for connecting a sample container collection device (403) to the blood sampling device, the blood sampling device having a reservoir (405) for collecting a small blood sample for point-of-care testing.

5. The intravenous system of claim 4, wherein the blood sampling device and the sample container collection device form a fluid path for blood flow into a sample container when the sample container is inserted into the sample container collection device.

6. The intravenous system of claim 1 or claim 4, wherein the kink resistant feature comprises an antimicrobial coating (303).

7. The intravenous system of claim 4, further comprising:
an antimicrobial lubricant applied to the diffuser tip.

8. The intravenous system of claim 4, wherein the catheter tubing comprises a water softening material and wherein the kink resistant feature also comprises a water softening material.

## Patentansprüche

1. Intravenöses System mit:
einem Katheteradapter (202);
einem Katheterschlauch (201), der von dem Katheteradapter ausgeht;
einem Knickresistenzmerkmal, das ein Knicken des Katheterschlauchs verhindert; und
eine Blutentnahmevorrichtung (401), die konfiguriert ist zur Abnahme einer kleinen Blutprobe für einen Point-of-care-Test und einer großen Blutprobe für einen Labortest;
**dadurch gekennzeichnet, dass**
die kleine Blutprobe in einem Reservoir (405) der Blutentnahmevorrichtung gesammelt wird und die große Blutprobe in einem Probenbehälter gesammelt wird, der über eine Probenbehälter-Sammelvorrichtung (403) mit der Blutentnahmevorrichtung verbunden ist.

2. Intravenöses System nach Anspruch 1, bei dem der Katheterschlauch ein Diffusor-Ende aufweist.

3. Intravenöses System nach Anspruch 2, bei dem das Diffusor-Ende eine antimikrobielle Beschichtung aufweist.

4. Intravenöses System nach Anspruch 2, bei dem
die Blutentnahmevorrichtung ein erstes Ende, das konfiguriert ist zum Verbinden der Blutentnahmevorrichtung mit dem Katheteradapter entweder direkt oder über ein Verlängerungs-Set (102), und ein zweites Ende aufweist, das einen Adapter zum Verbinden einer Probenbehälter-Sammelvorrichtung (403) mit der Blutentnahmevorrichtung bildet, wobei die Blutentnahmevorrichtung ein Reservoir (405) zum Aufnehmen einer kleinen Blutprobe für einen Point-of-care-Test aufweist.

5. Intravenöses System nach Anspruch 4, bei dem die Blutentnahmevorrichtung und die Probenbehälter-Sammelvorrichtung einen Fluidweg für einen Blutstrom in einen Probenbehälter bilden, wenn der Probenbehälter in die Probenbehälter-Sammelvorrichtung eingeführt ist.

6. Intravenöses System nach Anspruch 1 oder Anspruch 4, bei dem das Knickresistenzmerkmal eine antimikrobielle Beschichtung (303) aufweist.

7. Intravenöses System nach Anspruch 4, ferner mit:
einem antimikrobiellen Gleitmittel, das auf das Diffusor-Ende aufgebracht ist.

8. Intravenöses System nach Anspruch 4, bei dem der Katheterschlauch ein Wasserenthärtungsmaterial aufweist und bei dem das Knickresistenzmerkmal ebenfalls ein Wasserenthärtungsmaterial aufweist.

## Revendications

1. Système intraveineux comprenant :
un adaptateur de cathéter (202) ;
une tubulure de cathéter (201) s'étendant à partir de l'adaptateur de cathéter ;
un élément résistant au vrillage qui empêche le vrillage de la tubulure de cathéter ; et
un dispositif de prélèvement de sang (401) configuré pour collecter un petit échantillon de sang pour un test au point d'intervention et un grand échantillon de sang pour un test de laboratoire ;
**caractérisé en ce que**
le petit échantillon de sang est collecté dans un réservoir (405) du dispositif de prélèvement de sang, et le grand échantillon de sang est collecté dans un récipient d'échantillon relié au dispositif de prélèvement de sang par l'intermédiaire d'un dispositif de collecte de récipient d'échantillon (403).

2. Système intraveineux de la revendication 1, dans lequel la tubulure de cathéter a une pointe de diffusion.

3. Système intraveineux de la revendication 2, dans lequel la pointe de diffusion comporte un revêtement antimicrobien.

4. Système intraveineux de la revendication 2, dans lequel ledit dispositif de prélèvement de sang a une première extrémité qui est configurée pour relier le dispositif de prélèvement de sang à l'adaptateur de cathéter directement ou par l'intermédiaire d'un ensemble d'extension (102), et une deuxième extrémité formant un adaptateur pour relier un dispositif de collecte de récipient d'échantillon (403) au dispositif de prélèvement de sang, le dispositif de prélèvement de sang ayant un réservoir (405) pour collecter un petit échantillon de sang pour un test au point d'intervention.

5. Système intraveineux de la revendication 4, dans lequel le dispositif de prélèvement de sang et le dispositif de collecte de récipient d'échantillon forment un chemin de fluide pour l'écoulement de sang dans un récipient d'échantillon lorsque le récipient d'échantillon est inséré dans le dispositif de collecte de récipient d'échantillon.

6. Système intraveineux de la revendication 1 ou 4, dans lequel l'élément résistant au vrillage comprend un revêtement antimicrobien (303).

7. Système intraveineux de la revendication 4, comprenant en outre :
un lubrifiant antimicrobien appliqué à la pointe de diffusion.

8. Système intraveineux de la revendication 4, dans lequel la tubulure de cathéter comprend un matériau adoucisseur d'eau et dans lequel l'élément résistant au vrillage comprend également un matériau adoucisseur d'eau.
